# EUROPEAN PATENT SPECIFICATION

(11) **EP 0 637 452 B1**
(45) Date of publication and mention of the grant of the patent: **27.10.1999**
(21) Application number: 94112117.0
(22) Date of filing: 03.08.1994
(51) Int. Cl.: A61L 27/00, A61L 31/00, A61L 15/32

(54) **Collagen membrane material for medical use and process for preparing the same**
Kollagen Membranmaterial zur medizinischen Verwendung und Verfahren zu seiner Herstellung
Matériau à base de collagène pour membrane à usage médical et sa méthode de production

(30) Priority: 06.08.1993 JP 19575593; 01.09.1993 JP 21762893
(43) Date of publication of application: 08.02.1995
(73) Proprietor: Shimizu, Yasuhiko, Uji-shi, Kyoto-fu (JP)
(72) Inventor: Shimizu, Yasuhiko, Uji-shi, Kyoto-fu (JP)
(74) Representative: Hansen, Bernd, Dr. Dipl.-Chem.

(56) References cited:
- EP-A- 0 194 192
- WO-A-89/08467
- CA-A- 2 055 099
- UROLOGICAL RESEARCH, vol.15, no.1, February 1987 pages 53 - 59 GORHAM, S.D. 'THE IN VITRO ASSESSMENT OF A COLLAGEN/VICRYL (POLYGLACTIN) COMPOSITE FILM TOGETHER WITH CANDIDATE SUTURE MATERIALS FOR USE IN URINARY TRACT SURGERY.'
- JOURNAL OF BIOMEDICAL MATERIALS RESEARCH., vol.23, no.6, 1989 pages 649 - 660 JOHN A.M. RAMSHAW ET AL. 'COLLAGEN ORGANIZATION IN MANDREL-GROWN VASCULAR GRAFTS.'
- JOURNAL OF PEDIATRIC SURGERY, vol.28, no.5, May 1993 pages 660 - 663 R.N. MEDDINGS ET AL. 'A NEW BIOPROSTHESIS IN LARGE ABDOMINAL WALL DEFECTS.'
- UROLOGICAL RESEARCH, vol.16, no.5, 1988 pages 381 - 384 C.G. GEMMELL ET AL. 'THE IN VITRO ASSESSMENT OF A COLLAGEN/VICRYL (POLYGLACTIN) COMPOSITE FILM TOGETHER WITH CANDIDATE SUTURE MATERIALS FOR POTENTIAL USE IN URINARY TRACT SURGERY.'
- BRITISH JOURNAL OF UROLOGY, vol.68, no.4, October 1991 pages 421 - 424 R. SCOTT ET AL. 'FIRST CLINICAL REPORT OF A NEW BIODEGRADABLE MEMBRANE FOR USE IN UROLOGICAL SURGERY.'
- JOURNAL DE PARODONTOLOGIE, vol.10, no.3, 1991 pages 289 - 293 G. RACHLIN ET AL. 'BIOCOMPATIBILITE ET INTERETS D'UNE NOUVELLE MEMBRANE RESORBABLE (POLYGLACTIN 910-COLLAGENE) EN CHIRURGIE PARODONTALE.'
- ARTIFICIAL ORGANS, vol.6, no.3, August 1982, CLEVELAND (USA) pages 256 - 260 U.R. SHETTIGAR ET AL. 'COLLAGEN FILM FOR BURN WOUND DRESSINGS RECONSTITUTED FROM ANIMAL INTESTINES.'

## Description

### BACKGROUND OF THE INVENTION

The present invention relates to a material for medical use and a process for preparing the same, and more detailedly, a material for medical use which is applicable to an artificial organ and an artificial viscus, furthermore, which is applicable as a wound covering material, a wound prosthetic material, a wound curing material, a post-operative adhesion-preventing material and the like, and a process for preparing the same.

It has been known long that, when a living tissue has some disorders, is damaged, or has dysfunction, an artificial material is used for replacement, prosthesis, or prevention of adhesion of damaged parts, and it has been conventionally investigated to use a synthetic high molecular material or a material originating from a living body for vessels, air tubes, esophagi, valves, various viscera, and wounds. Such a material for medical use is required to satisfy various requirements that it has an affinity for a living body; it has a compatibility with body fluids such as blood, or tissues; it has neither toxicity nor antigenicity; and it has a mechanical strength as predetermined depending on the implant site.

Generally, a material originating from a living body may possibly cause disorders associated with implantation or immunological responses, but a collagen, a material originating from a living body, is excellent in affinity for a living body and tissue-compatibility, possesses low antigenicity, a facilitating activity on elongation and proliferation of host cells, as utilized as a medium for cell culture, and a styptic activity. It also has an excellent property as a material for medical use since it is completely absorbed in a living body. However, when the collagen only is used, it is difficult to form therefrom a material which has a high invading ability into cells and a high proliferating ability for host cells, and also has a considerable mechanical strength. Therefore, the collagen has been conventionally used as a composite material with a synthetic high molecular material.

Such a composite material for medical use is produced by forming a covering layer of collagen comprising an alkaline-solubilized collagen or an enzyme-solubilized collagen, having reduced antigenicity, on a surface of the synthetic high molecular material comprising a silicone, a polytetrafluoroethylene, a polyethylene, a polypropylene, a polyethylene terephthalate, a polyurethane, a polyvinyl alcohol and a nylon in the form of film, sheet, woven fabric, non-woven fabric, tube, sponge and the like, by a coating or a flowing into method and by solidifying the covering layer of collagen by a freeze-drying method and the like. The surface of the synthetic high molecular material is subjected to a hydrophilization treatment by plasma irradiation and the like, so as to improve the affinity for living tissue or the covering layer of collagen.

Another material for medical use has been proposed wherein a material degradable in a living body, having a considerable mechanical strength, which is hydrolyzed or enzymatically decomposed and absorbed in a living body, is combined with collagen.

The material for medical use is produced by solidifying a covering layer comprising an alkaline-solubilized collagen or an enzyme-solubilized collagen, by the above method, on a surface of a material degradable in a living body in the form of film, sheet, woven fabric, or non-woven fabric, comprising a polyglycolic acid, a copolymer of glycolic acid and lactic acid, or a mixture of a polyglycolic acid and a polylactic acid. When the material for medical use is implanted in a living body, not only collagen but also the degradable material is hydrolyzed or enzymatically decomposed, and absorbed in the living body, so that it is not necessary to remove the material by conducting an operation again or an endoscopy. The material degradable in a living body is also subjected to a hydrophilization treatment with plasma irradiation as well as the synthetic high molecular material.

However, such a material for medical use has a disadvantage in the transfixing property that, when an intermediary material comprising a material degradable in a living body in the form of woven fabric or non-woven fabric, equipped with a covering layer of collagen, is sutured with an organ or a wound, a needle for operation cannot easily pass through a hole, or even if the needle can pass through the hole, force added to the covering layer of collagen may destroy the covering layer of collagen having a small mechanical strength. This material has another disadvantage that although the affinity for a collagen solution is improved by plasma irradiation on the surface of the intermediary material, the covering layer of collagen may peel from the intermediary material after the implantation because of the insufficient adhesivity between the intermediary material and the covering layer of collagen, and in such a case, it is required to conduct a operation again to implant the material.

Urological Research, Vol. 15, No. 1, February 1, 1987, pages 53 to 59 and EP-A-0 194 192 disclose surgical reinforcement materials made from two sheets of collagen which are separated by a mesh-like material. They do not make use of an adhesive to join the layers.

### SUMMARY OF THE INVENTION

The present invention was developed in order to solve these problems, and an object of the present invention is to provide a material for medical use which is applicable to an artificial organ or an artificial viscus, and further applicable as a wound covering material, a wound prosthetic material, a wound curing material, a post-operative adhesion-preventing material and the like, is excellent in affinity for a living body, tissue compatibility, and mechanical strength, possesses low antigenicity, and is particularly excellent in transfixing property; and is also to provide a process for preparing the same.

The material for medical use of the present invention comprises two sheets of collagen membrane adhered to each other with an adhesive and having interposed therebetween a mesh-like intermediary material, wherein the mean pore diameter of the mesh-like intermediary material is between 100 and 2000 µm. The process for preparing the same comprises laminating with an adhesive two sheets of collagen membrane having a mesh-like intermediary material interposed therebetween, wherein the mean pore diameter of the mesh-like intermediary material is between 100 and 2000 µm; maintaining the resulting laminated material under a reduced pressure so that the two sheets of collagen membrane are adhered to each other; and cross-linking the laminated material. Furthermore, the process may comprise subjecting the laminated material to a succinylation treatment, depending upon the purpose.

### DESCRIPTION OF THE PREFERRED EMBODIMENTS

In the following, the present invention is explained in detail.

As collagen usable as the material of the collagen membrane of the present invention, various collagens which have been usually used can be used, and there can be mentioned, for example, a neutral solubilized collagen, an acid-solubilized collagen, an alkali-solubilized collagen, an enzyme-solubilized collagen and the like. Among them, the alkali-solubilized collagen and the enzyme-solubilized collagen which are obtained by treating an insoluble collagen with an alkali and an enzyme such as pepsin, trypsin, chymotrypsin, papain and pronase, respectively, can be preferably used because a telopeptide site in the collagen molecule, having a high antigenicity, is removed by the treatment and therefore the antigenicity is lowered.

The source of the collagen is not particularly specified; collagens derived from skin, bone, cartilage, tendon, organ and the like of mammalian animals such as cow, pig, rabbit sheep, mouse and the like, can be generally used. A collagen-like protein derived from fishes and birds can be also used.

When an alkali-solubilized collagen or an enzyme-solubilized collagen is used as the collagen membrane, a collagen solution layer is formed using a conventional method such as a coating method and a flowing into method, and then, the collagen membrane is formed by means for freeze-drying and the like. The thickness of the collagen solution layer is regulated so that the finally formed collagen membrane has a thickness of 2 mm to 20 mm, preferably 5 mm to 10 mm. If the thickness of the collagen membrane is less than 2 mm, the collagen is rapidly absorbed in a living body, resulting in the insufficient effects. If the thickness is 20 mm or more, no notable problem will be caused, but some problem may be caused in the viewpoint of the operation. The collagen membrane is preferably formed into a porous membrane so that, when the membrane is implanted into a living body, cells can easily invade, elongate, and proliferate in the membrane. The concentration of the collagen solution, used here, which can be properly regulated depending on the thickness and the density of required collagen membrane, is 0.1 to 5 % by weight, preferably 0.5 to 2 % by weight. When the collagen membrane is formed into a porous membrane, a collagen solution bubbled by stirring is used.

The above freeze-drying procedure can be carried out according to a conventional method. In such a case, the collagen is desirably fiberized prior to the freeze-drying procedure so as to enhance the strength of the collagen membrane. The fiberizing procedure can be carried out by changing the hydrogen ion concentration of the collagen solution, or by elevating the temperature thereof.

Furthermore, a purified collagen derived from a living body can be used as it is, as the material of the collagen membrane, and a purified human amnion or a purified human chorion obtained from a human placenta is preferred. Such a material contains human collagen as a main component, so that it has low antigenicity, procedures such as solubilization and coating can be omitted, and it has a suitable strength.

The purified human amnion or the purified human chorion can be used as the collagen membrane in the manner as described in, for example, Japanese Laid-Open Patent Application (KOKAI) No. 56987/1993. Namely, only a human fetal membrane is separated from a mass comprising human fetal membrane, placenta and umbilical cord, collected immediately after labor in a 1 % benzalkonium chloride solution or benzalkonium bromide solution, and an amnion or a chorion, which is the substrate type V collagen membrane, is peeled from the fetal membrane consisting of 4 layers. Then, remaining tissues and the like are physically or enzymatically removed, followed by washing with ultrasonic wave to have a purified human amnion or human chorion.

A human amnion has a structural difference between the two surfaces. One surface facing toward a fetus is smooth and comprises fine fibers having a thickness of 0.1 µm or less and cells hardly adhere to it. Another surface facing toward a chorion which is rough and comprises thick fasciculus having a thickness of 0.5 to 0.2 µm is suitable for cell proliferation and take. Therefore, when the material for medical use of the present invention is used as an adhesion-preventing material, the mesh-like intermediary material is preferably interposed with two sheets of the collagen membrane of a purified human amnion so that the surfaces thereof facing toward a fetus are outside. On the other hand, if the material for medical use of the present invention is used for the purpose of cell proliferation or take, the mesh-like intermediary material is preferably interposed with two sheets of the collagen membrane of a purified human amnion so that the surfaces thereof facing toward a chorion are outside.

The above-described collagen membrane is subjected to a cross-linking treatment. The cross-linking treatment is carried out in order to fix the two sheets of collagen membrane closely adhered to each other in an integrated form, through an adhesive, described below, and also regulate the decomposition/absorption rate of the collagen membrane, depending on the intended use of the material for medical use of the present invention. Namely, by properly changing the reaction conditions of the cross-linking treatment to have a collagen membrane neither degradable nor absorbable in a living body or a collagen membrane degradable and absorbable in a living body, and furthermore by combining the collagen membrane with a mesh-like intermediary material comprising a material neither degradable nor absorbable in a living body or a material degradable and absorbable in a living body, described later, various kinds of material for medical use, applicable to an artificial organ and an artificial viscus, or applicable as a wound-covering material, a wound-prosthetic material, a wound-curing material, and the like, can be obtained.

For example, by combining a collagen membrane degradable and absorbable in a living body, with a mesh-like intermediary material comprising a material degradable and absorbable in a living body, the resulting material can be used as a suture-reinforcing material, a prosthetic material or an artificial organ as replaced with tissues of the living body in the process of regeneration. Also, by combining a collagen membrane degradable and absorbable in a living body with a mesh-like intermediary material comprising a material neither degradable nor absorbable in a living body, the resulting material can be used as a prosthetic material or an artificial organ which is required to permanently maintain the tissue strength of the living body after the collagen membrane is replaced by living tissues. By combining a collagen membrane non-degradable in a living body with a material neither degradable nor absorbable in a living body, the resulting material can be applied to an artificial organ such as an artificial valve which is required only to have a molding-processability and not to permanently react with living tissues at all.

A process for the cross-linking treatment may include a process by a glutaraldehyde cross-linking, an epoxy cross-linking or a heat cross-linking. The glutaraldehyde cross-linking can be carried out by immersing the material in a glutaraldehyde solution having a concentration of 0.05 to 3 %, preferably 0.1 to 2 %, followed by air-drying. If the concentration is less than 0.05 %, the collagen membrane easily peels off. If the concentration is over 3 %, the collagen membrane is hardened, resulting in the loss of the affinity for a living body. The glutaraldehyde cross-linking is presumed to proceed with a reaction of an amino group in the collagen molecule with an aldehyde group of glutaraldehyde.

The epoxy cross-linking can be carried out by immersing the material in a solution comprising an epoxy compound having 2 epoxy groups in a molecule, a hardening-accelerator, and the like. For example, the material is immersed in 2 % DENACOLE solution having a pH value of 10, prepared by adding 47.5 ml of 0.1 M carbonate buffer and 47.5 ml of ethanol to 5 ml of original DENACOLE solution (manufactured by Nagase Industries), a hydrophilic cross-linking agent, for 12 to 24 hours at a room temperature, washed in water well, and air-dried. The epoxy cross-linking is presumed to proceed with a reaction of an amino group in the collagen molecule with an epoxy group in the epoxy compound.

The heat cross-linking can be carried out by heating the material in vacuo at 90 to 200 °C, preferably 105 to 150 °C, and dehydrating it to cause a cross-linking reaction. The time for heating the material, which may be properly regulated depending on the heating temperature, the degree of reduced pressure, and the desired degree of cross-linking, is usually 6 to 24 hours. The heat cross-linking of collagen is presumed to proceed with a formation of Schiff base or an aldol condensation between a sugar chain in the collagen molecule or an aldehyde group resulting from oxidization and lysine or hydroxylysine in the collagen molecule. In consideration of these points, as a collagen membrane comprising an alkali-solubilized collagen or an enzyme-solubilized collagen, a collagen originating from pig is preferred because it, having a high content of sugar chain, easily forms the cross-linking structure.

The mesh-like intermediary material imposed between the above collagen membranes may be preferably in the form of, for example, mesh sheet, woven fabric, non-woven fabric, or sheet having pores formed, and the mean pore diameter is 100 to 2000 µm from the viewpoint of the degradability or elasticity in a living body, or so that the collagen membranes can be closely adhered to each other through pores of the mesh-like intermediary material. If the mean diameter is less than 100 µm, the collagen membranes are hardly adhered closely, and if the mean pore diameter is over 2000 µm, cracks will form in the collagen membranes when sutured, resulting in the low transfixing property. The mean pore diameter is preferably 100 to 1500 µm, more preferably 150 to 1000 µm, most preferably 150 to 500 µm. The thickness is preferably 100 to 1000 µm. In a case of a non-woven fabric, the pore diameter differs each other, and therefore the mean pore diameter mentioned in the present invention is a mean value calculated by regarding the diameter of a circle having an area being the same as that of the pore as the pore diameter.

As the mesh-like intermediary material, a material degradable in a living body or a material non-degradable in a living body can be used. Namely, if it is intended to implant the material for medical use of the present invention into a living body, a material degradable in a living body can be used. If it is intended to permanently maintain the strength of regenerating tissues, or to apply the material to an artificial skin, a material non-degradable in a living body can be used.

As the material degradable in a living body, various kinds of material can be used as far as it is degraded through hydrolysis or enzymolysis, and absorbed in a living body, and it has no toxicity and has a mechanical strength to a some extent. A polyglycolic acid, a polylactic acid, a copolymer of glycolic acid and lactic acid, a polydioxanone, a copolymer of glycolic acid and trimethylene carbonate, a mixture of a polyglycolic acid and a polylactic acid, and the like can be preferably used.

As the material non-degradable in a living body, a synthetic high molecular compound such as a silicone, a polytetrafluoroethylene, a polyethylene, a polypropylene, a polyethylene terephthalate, a polyurethane, a polyvinyl alcohol, and a nylon which have been usually used, can be used.

The mesh-like intermediary material is preferably subjected to a hydrophilization treatment by plasma irradiation so as to improve the affinity for living tissues or the collagen membrane.

As the adhesive which adheres the above collagen membranes directly each other, a gelatin solution or a collagen solution can be preferably used; as a gelatin for a raw material of the gelatin solution for the adhesive, purified gelatin listed in the Pharmacopoeia of Japan, can be generally used. As a collagen for a raw material of the collagen solution, an acid-solubilized collagen, an alkali-solubilized collagen and an enzyme-solubilized collagen can be used. The concentration of the gelatin solution is 1.0 to 5.0 % by weight, preferably 2.0 to 3.0 % by weight. The concentration of the collagen solution is 0.5 to 3.0 % by weight, preferably 1.0 to 3.0 % by weight.

The process for preparing the material for medical use of the present invention is explained below.

As the first step, the above mesh-like intermediary material is subjected to a plasma discharge treatment, then immersed in or coated with an adhesive comprising the above gelatin solution or the above collagen solution. Then, the mesh-like intermediary material is interposed by two sheets of the above collagen membrane to laminate them. If a purified human amnion is used as the collagen membrane, the mesh-like intermediary material is interposed so that required side of the surfaces of the amnion is outside, depending on the purpose.

As the second step, the thus obtained laminated material is kept under reduced pressure of 10 to 20 Torr at a room temperature for about 0.1 hour to remove an air between the two sheets of collagen membrane or between the mesh-like intermediary material and the collagen membrane so as to closely adhere the two sheets of the collagen membrane to each other, and the laminated material is simultaneously dried. The second step can be carried out by air-drying only without the procedures under reduced pressure.

As the third step, the laminated material which had been dried under reduced pressure, is subjected to a glutaraldehyde cross-linking, an epoxy cross-linking or a heat cross-linking to fix the collagen membranes in an integrated form and simultaneously make the laminated material neither degradable nor absorbable in a living body, or degradable and absorbable in a living body, depending on the intended use.

When required, the obtained laminated material can be immersed in a glycerin solution and the like to afford a flexibility to the collagen membranes.

Through the above described steps, the material for medical use of the present invention comprising two sheets of collagen membrane adhered to each other with an adhesive and having interposed therebetween a mesh-like intermediary material can be obtained, and is applicable to an artificial organ or an artificial viscus, and applicable as a wound covering material, a wound prosthetic material, a wound curing material and the like.

Furthermore, the material for medical use of the present invention can be applicable as an adhesion-preventing material by subjecting the material for medical use of the present invention wherein the collagen membrane comprises a material degradable and absorbable in a living body, to a succinylation treatment, causing most of amino groups, remaining in the collagen molecule to react with succinic anhydride. This succinylation can be carried out according to a usual method, and for example, the material for medical use of the present invention is immersed in a mixture comprising 250 ml of 0.02 M borate buffer having a pH value of 9.0 and 50 ml of a 5 % succinic anhydride solution in acetone for 1 to 48 hours, preferably 12 to 24 hours, washed with water, and dried under reduced pressure.

In order to confirm that the collagen membrane is succinylated, the material for medical use which has been succinylated is immersed in , for example, a 0.33 % ninhydrin aqueous solution for 3 to 5 minutes. Ninhydrin coloring reaction is based on a condensation product resulting from a reaction of an amino group in the collagen molecule with ninhydrin, and as the collagen membrane is more succinylated, the membrane becomes non-dyeable.

### EXAMPLES

The present invention will be more detailedly explained with references to the following Examples, Comparative examples and Test examples.

### Examples 1 and 2, and Comparative Examples 1 through 4

As two kinds of mesh-like intermediary material comprising a polyglycolic acid, DEXON MESH (manufactured by Nihon Lederle Co.), PGA MESH (manufactured by Gunze Co.), and TGP 1800 comprising a polyethylene terephthalate (manufactured by Gunze Co.) were obtained, and, on a sheet of aluminium foil having the same size as that of the above intermediary material, were hydrophilized by a discharge treatment for 10 minutes by using a Tesla coil. Then, it was immersed in a 2.0 % gelatin solution or a 1.0 % collagen solution. The mean pore diameter of the mesh-like intermediary material is 50 µm for DEXON MESH, 200 µm for PGA MESH, and 10 µm for TGP 1800. As the gelatin, purified gelatin listed in the Pharmacopoeia of Japan, and as the collagen, type I collagen obtained from pig were used.

An amnion was peeled from a human fetal membrane, and foreign matters were removed from the amnion. Then the amnion was subjected to a ficin treatment by immersing it in 0.01 % ficin solution in 0.2 M phosphate buffer, having a pH value of 7.4, and was subjected to an ultrasonic treatment in purified water so as to completely remove foreign matters. And, the amnion was immersed in a benzalkonium chloride aqueous solution, and using 2 sheets of the thus obtained purified human amnion, the mesh-like intermediary material after immersed in the collagen solution was interposed therebetween for lamination.

The obtained laminated material was dried by allowing it to stand in a desiccator which employs phosphorus pentoxide as a drying agent at a room temperature for 15 hours. After drying, the desiccator was connected with a vacuum pump, and aspirated for 10 minutes to cause the two sheets of the purified human amnion to closely adhere to each other.

Then, the thus obtained laminated material was placed in a thermostatic bath together with the desiccator, the temperature of the bath was set at 105 °C in vacuo, and the laminated material was allowed to stand for 24 hours after the temperature began to elevate so as to cause a cross-linking reaction. Furthermore, the laminated material was immersed in a 5 % glycerin solution for 30 minutes to afford a flexibility to the purified human amnion, and dried in a desiccator under reduced pressure to give the material for medical use.

### Examples 3 through 6

A material for medical use was prepared by using PGA MESH having a mean pore diameter of 200 µm as used in Example 1, in the same manner as in Example 1 except that a cross-linking treatment was carried by immersing the laminated material in 0.05 %, 0.1 %, 0.5 %, and 1.0 % glutaraldehyde solutions for 1 hour in place of the heat cross-linking treatment.

### Comparative Examples 5 and 6

As the mesh-like intermediary material, PGA MESH having a mean pore diameter of 200 µm, used in Example 1, was used, and the both surfaces thereof were treated by using an apparatus for plasma irradiation for 5 minutes, respectively, for hydrophilization.

Fifty ml of a 1 % solution of an enzyme-solubilized collagen originating from porcine skin, having a pH value of 3.0, or 50 ml of a 1 % solution of an alkali-solubilized collagen originating from porcine skin, having a pH value of 9.0, was stirred with a stirrer at 3000 rpm for 5 minutes to bubble it. Both surfaces of PGA MESH were coated with the solution, and subjected to a neutralization treatment under an ammonia atmosphere for 30 minutes so as to gelatinize the collagen. After the neutralization treatment, the resulting material was well washed with distilled water to remove ammonia, and immediately freeze-dried to have the PGA MESH coated with porous collagen layer. After freeze-drying, the material was furthermore subjected to a heat treatment at 105 °C for 12 hours in vacuo to have a material for medical use.

### Example 7

The material for medical use prepared in Example 1 was immersed in a mixed solution comprising 250 ml of 0.02 M borate buffer having a pH value of 9.0 and 50 ml of 5 % succinic anhydride solution in acetone at a room temperature for 24 hours, washed with water, and air-dried overnight for succinylization.

### Comparative Example 7

A polyglycolic acid yarn of 12 filament and 35 denier, obtained by a fusion-preventing method was treated with heat at 106 °C for 3 hours, and knitted with an apparatus for tubularly knitting to have a tubular plain fabric. The obtained fabric was 4-fold laminated and subjected to needle-punching so as to have a polyglycolic acid non-woven fabric wherein interstices can be substantially invisible. The fabric was subjected to a heat press at 100 °C for 5 minutes to remove the hairiness or frays. The polyglycolic acid non-woven fabric was cut into pieces 10 cm in size, and both surfaces thereof were treated with a plasma irradiation apparatus for 5 minutes for hydrophilization.

Fifty ml of a 1 % solution of enzyme-solubilized collagen originating from porcine skin, having a pH value of 3.0 was stirred with a stirrer at 3000 rpm for 5 minutes to bubble it, applied to one surface of the above polyglycolic acid non-woven fabric, and subjected to a neutralization treatment for 30 minutes under an ammonia atmosphere to gelatinize the collagen. After the neutralization treatment, the resulting material was well washed in distilled water to remove ammonia, and immediately freeze-dried to have the polyglycolic acid non-woven fabric coated with porous collagen layer. After freeze-drying it, it was treated with heat at 105 °C in vacuo for 12 hours to have a material for medical use.

### Test Example 1

The peel strength of the material for medical use, prepared in Examples 1 through 7 and Comparative examples 1 through 7 was determined under dry conditions. For measurement of the peeling strength of the collagen membrane, the material for medical use was cut into pieces, 1 cm in size, to have samples for the test, and one surface of the sample was adhered to a floor face, and another surface was adhered to a plastic plate equipped with a thread. The thread was connected with a strain gauge, and the thread was pulled until the collagen membrane peeled off so as to determine the peel strength of the collagen membrane.

Also, the material for medical use was immersed in a physiological saline at 37 °C to determine whether the collagen membrane peeled off. The results are shown in Table 1.

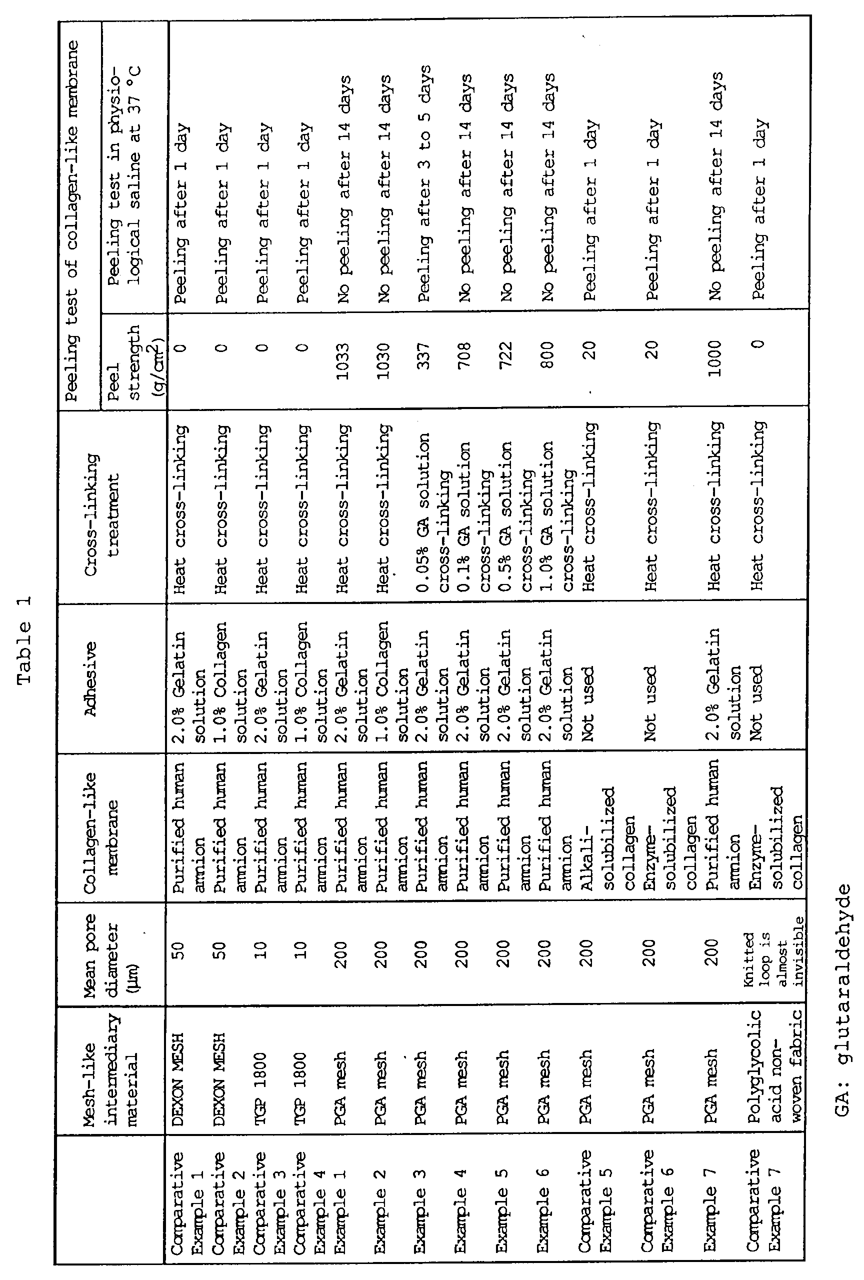

### Test Example 2

In order to confirm the usefulness of the material for medical use of the present invention, the materials for medical use, prepared in Examples 1 and 2, Examples 4 through 6 and Comparative examples 1 through 6, 1 cm in size, were sutured to lung, heart, intestine and muscle of a rabbit, weighing 3 kg, and a dog, weighing 15 kg, using polypropylene suture. As a result, in the case of the materials for medical use prepared in Examples 1 and 2 and Examples 4 through 6, a needle for operation could be easily passed through pores of the mesh-like intermediary materials, and the collagen membrane was not destroyed. But, in the case of Comparative examples 1 through 6, the collagen membrane was destroyed.

Also, a part of pericardium, 5 cm in size, of a dog, weighting 15 kg, was resected, and the resected part was replaced with the material for medical use prepared in Example 7.

When the operation was carried out again after 6 weeks, the pericardium regenerated without adhesion.

A part of oviduct muscle, 2 cm in size, and a part of peritoneum, 2 cm in size, of a rabbit weighing 3 kg, were resected, and the materials for medical use prepared in Examples 4 through 6 and Example 7 were inserted into the resected site. When the operation was carried out again after 3 weeks, the oviduct and the peritoneum did not show adhesion.

The material for medical use of the present invention is excellent in affinity for a living body and tissue-compatibility, and possesses low antigenicity because a collagen membrane is employed. Since the mean pore diameter of the mesh-like intermediary material is as large as 100 to 2000 µm, a needle for operation can be easily passed through the pore when it is sutured to an organ or a trauma part. Since the mesh-like intermediary material is interposed between the collagen membranes, and the mechanical strength of the collagen membrane is enhanced by a cross-linking treatment, the collagen membrane is not easily destroyed when a needle for operation is passed through it. Thus, it is excellent in transfixing property. Also, since two sheets of the collagen membrane, closely adhered to each other through an adhesive, are fixed in an integrated form by a cross-linking treatment, the collagen membrane does not peel off and it is not required to carry out an operation again for re-implantation, when the material for medical use is implanted into a living body.

Furthermore, the collagen membrane is made degradable and absorbable in a living body, or made neither degradable nor absorbable in a living body by subjecting the collagen membrane to a cross-linking treatment. Therefore, by combining the collagen membrane with the mesh-like intermediary material comprising a material degradable and absorbable in a living body or a material non-degradable in a living body, the material for medical use of the present invention can be used as various materials for medical use.

## Claims

1. A material for medical use comprising two sheets of collagen membrane adhered to each other with an adhesive and having interposed therebetween a mesh-like intermediary material, wherein the mean pore diameter of the mesh-like intermediary material is between 100 and 2000 µm.

2. The material of Claim 1, wherein the collagen membrane is a collagen solubilized with an alkali or a collagen solubilized with an enzyme.

3. The material of Claim 1, wherein the collagen membrane is of a human amnion or a human chorion.

4. The material of Claim 3, wherein the mesh-like intermediary material is interposed with two sheets of the collagen membrane of a human amnion so that the surfaces thereof facing toward a fetus are outside.

5. The material of Claim 3, wherein the mesh-like intermediary material is interposed with two sheets of the collagen membrane of a human amnion so that the surfaces thereof facing toward a chorion are outside.

6. The material of Claim 1, wherein the mean pore diameter of the mesh-like intermediary material is 100 to 1500 µm.

7. The material of Claim 1, wherein the mesh-like intermediary material is degradable and absorbable in a living body.

8. The material of Claim 1, wherein the mesh-like intermediary material is not degradable and not absorbable in a living body.

9. The material of Claim 7, wherein the intermediary material is a poly(glycolic acid), a poly(lactic acid), a copolymer of glycolic acid and lactic acid, a polydioxanone, a copolymer of glycolic acid and trimethylene carbonate or a mixture of a poly(glycolic acid) and a poly(lactic acid).

10. The material of Claim 8, wherein the intermediary material is neither degradable nor absorbable in a living body and is a silicone, a polytetrafluoroethylene, a polyethylene, a polypropylene, a polyethylene terephthalate, a polyurethane, a polyvinyl alcohol or a nylon.

11. The material of Claim 1, wherein the adhesive is a collagen solution or a gelatin solution.

12. A process for preparing a material for medical use comprising:
(a) laminating with an adhesive two sheets of collagen membrane having a mesh-like intermediary material interposed therebetween, wherein the mean pore size of the mesh-like intermediary material is between 100 and 2000 µm;
(b) maintaining the resulting laminated material under a reduced pressure so that the two sheets of collagen membrane are adhered to each other through the mesh-like intermediary; and
(c) cross-linking the laminated material.

13. The process of Claim 12, wherein the material is subjected further to succinylation treatment.

14. The process of Claim 12, wherein the collagen membrane is a collagen solubilized with an alkali or a collagen solubilized with an enzyme.

15. The process of Claim 12, wherein the collagen membrane is of a human amnion or a human chorion.

16. The process of Claim 15, wherein the mesh-like intermediary material is interposed with two sheets of the collagen membrane of a human amnion so that the surfaces therof facing toward a fetus are outside.

17. The process of Claim 15, wherein the mesh-like intermediary material is interposed with two sheets of the collagen membrane of a human amnion so that the surfaces thereof facing toward a chorion are outside.

18. The process of Claim 12, wherein the mean pore diameter of the mesh-like intermediary material is 100 to 2000 µm.

19. The process of Claim 12, wherein the mesh-like intermediary material is degradable and absorbable in a living body.

20. The process of Claim 12, wherein the mesh-like intermediary material is not degradable and not absorbable in a living body.

21. The process of Claim 19, wherein the intermediary material is a poly(glycolic acid), a poly(lactic acid), a copolymer of glycolic acid and lactic acid, a polydioxanone, a copolymer of glycolic acid and trimethylene carbonate or a mixture of a poly(glycolic acid) and a poly(lactic acid).

22. The process of Claim 20, wherein the intermediary material is a silicone, a polytetrafluoroethylene, a polyethylene, a polypropylene, a polyethylene terephthalate, a polyurethane, a polyvinyl alcohol or a nylon.

23. The process of Claim 12, wherein the adhesive is a collagen solution or a gelatin solution.

## Patentansprüche

1. Material zur medizinischen Verwendung, umfassend zwei Folien einer Kollagenmembran, die aneinander mit einem Klebemittel verklebt sind, mit einem dazwischengeschobenen siebartigen Zwischenmaterial, worin der mittlere Porendurchmesser des siebartigen Zwischenmaterials zwischen 100 und 2000 µm liegt.

2. Material des Anspruchs 1, worin die Kollagenmembran mit einem Alkali solubilisiertes Kollagen oder mit einem Enzym solubilisiertes Kollagen ist.

3. Material des Anspruchs 1, worin die Kollagenmembran von humanem Amnion oder humanen Chorion stammt.

4. Material des Anspruchs 3, worin das siebartige Zwischenmaterial zwischen zwei Folien der Kollagenmembran von humanem Amnion so eingeschoben ist, daß ihre gegen einen Fetus gerichteten Oberflächen außen liegen.

5. Material des Anspruchs 3, worin das siebartige Zwischenmaterial zwischen zwei Folien der Kollagenmembran von humanem Amnion so eingeschoben ist, so daß ihre gegen ein Chorion gerichteten Oberflächen außen liegen.

6. Material des Anspruchs 1, worin der mittlere Porendurchmesser des siebartigen Zwischenmaterials 100 bis 1500 µm beträgt.

7. Material des Anspruchs 1, worin das siebartige Zwischenmaterial im lebenden Körper abbaubar und absorbierbar ist.

8. Material des Anspruchs 1, worin das siebartige Zwischenmaterial im lebenden Körper nicht abbaubar und nicht absorbierbar ist.

9. Material des Anspruchs 7, worin das siebartige Zwischenmaterial eine Polyglycolsäure, eine Polymilchsäure, ein Copolymer von Glycolsäure und Milchsäure, ein Polydioxanon, ein Copolymer von Glycolsäure und Trimethylencarbonat oder eine Mischung von Polyglycolsäure und Polymilchsäure ist.

10. Material des Anspruchs 8, worin das Zwischenmaterial im lebenden Körper weder abbaubar noch absorbierbar ist und ein Silicon, ein Polytetrafluorethylen, ein Polyethylen, ein Polypropylen, ein Polyethylenterephthalat, ein Polyurethan, ein Polyvinylalkohol oder ein Nylon ist.

11. Material gemäß Anspruch 1, worin das Klebemittel eine Kollagenlösung oder eine Gelatinelösung ist.

12. Verfahren zur Herstellung eines Materials zur medizinischen Verwendung, umfassend
(a) Laminieren von zwei Folien einer Kollagenmembran mit einem dazwischengeschobenen siebartigen Zwischenmaterial, worin der mittlere Porendurchmesser des siebartigen Zwischenmaterials zwischen 100 und 2000 µm liegt, mit einem Klebemittel;
(b) Halten des resultierenden laminierten Materials unter einem vermindertem Druck, so daß die zwei Folien der Kollagenmembran über die siebartige Zwischenschicht aneinanderhaften; und
(c) Vernetzung des laminierten Materials.

13. Verfahren des Anspruchs 12, worin das Material ferner einer Succinylierungsbehandlung unterworfen wird.

14. Verfahren des Anspruchs 12, worin die Kollagenmembran ein mit einem Alkali solubilisiertes Kollagen oder ein mit einem Enzym solubilisiertes Kollagen ist.

15. Verfahren des Anspruchs 12, worin die Kollagenmembran aus einem humanem Anion oder einem humanem Chorion besteht.

16. Verfahren des Anspruchs 15, worin das siebartige Zwischenmaterial so zwischen zwei Folien der Kollagenmembran aus einem humanem Amnion eingeschoben ist, daß ihre gegen einen Fetus gerichteten Oberflächen außen liegen.

17. Verfahren des Anspruchs 15, worin das siebartige Zwischenmaterial zwischen zwei Folien der Kollagenmembran aus einem humanem Amnion eingeschoben ist, daß ihre gegen ein Chorion gerichteten Oberflächen außen liegen.

18. Verfahren des Anspruchs 12,, worin der mittlere Porendurchmesser des siebartigen Zwischenmaterials 100 bis 2000 µm beträgt.

19. Verfahren des Anspruchs 12, worin das siebartige Zwischenmaterial im lebenden Körper abbaubar und absorbierbar ist.

20. Verfahren des Anspruchs 12, worin das siebartige Zwischenmaterial im lebenden Körper nicht abbaubar und nicht absorbierbar ist.

21. Verfahren des Anspruchs 19. worin das siebartige Zwischenmaterial eine Polyglycolsäure, eine Polymilchsäure, ein Copolymer von Glycolsäure und Milchsäure, ein Polydioxanon, ein Copolymer von Glycolsäure und Trimethylencarbonat oder eine Mischung von Polyglycolsäure und Polymilchsäure ist.

22. Verfahren des Anspruchs 20, worin das Zwischenmaterial ein Silicon, ein Polytetrafluorethylen, ein Polyethylen, ein Polypropylen, ein Polyethylenterephthalat, ein Polyurethan, ein Polyvinylalkohol oder ein Nylon ist.

23. Verfahren des Anspruchs 12, worin das Klebemittel eine Kollagenlösung oder eine Gelatinelösung ist.

## Revendications

1. Matériau destiné à usage médical comprenant deux feuilles de membrane de collagène adhérant l'une à l'autre avec un adhésif et ayant interposé entre elles un matériau intermédiaire analogue à un treillis, dans lequel le diamètre moyen de pores du matériau intermédiaire analogue à un treillis est compris entre 100 et 2 000 µm.

2. Matériau selon la revendication 1, dans lequel la membrane de collagène est un collagène solubilisé avec une base ou un collagène solubilisé avec une enzyme.

3. Matériau selon la revendication 1, dans lequel la membrane de collagène provient d'un amnios humain ou d'un chorion humain.

4. Matériau selon la revendication 3, dans lequel le matériau intermédiaire analogue à un treillis est interposé deux feuilles de la membrane de collagène d'un amnios humain de sorte que leurs surfaces dirigées vers un foetus se trouvent à l'extérieur.

5. Matériau selon la revendication 3, dans lequel le matériau intermédiaire analogue à un treillis est interposé avec deux feuilles de la membrane de collagène d'un amnios humain de sorte que leurs surfaces dirigées vers un chorion se trouvent à l'extérieur.

6. Matériau selon la revendication 1, dans lequel le diamètre moyen de pores du matériau intermédiaire analogue à un treillis est de 100 à 1 500 µm.

7. Matériau selon la revendication 1, dans lequel le matériau intermédiaire analogue à un treillis est dégradable et absorbable dans un corps vivant.

8. Matériau selon la revendication 1, dans lequel le matériau intermédiaire analogue à un treillis n'est pas dégradable et n'est pas absorbable dans un corps vivant.

9. Matériau selon la revendication 7, dans lequel le matériau intermédiaire est un poly(acide glycolique), un poly(acide lactique), un copolymère de l'acide glycolique et de l'acide lactique, une polydioxanone, un copolymère de l'acide glycolique et du carbonate de triméthylène ou un mélange d'un poly(acide glycolique) et d'un poly(acide lactique).

10. Matériau selon la revendication 8, dans lequel le matériau intermédiaire n'est ni dégradable ni absorbable dans un corps vivant et est un silicone, un polytétrafluoroéthylène, un polyéthylène, un polypropylène, un poly(téréphtalate d'éthylène), un polyuréthanne, un poly(alcool vinylique) ou un nylon.

11. Matériau selon la revendication 1, dans lequel l'adhésif est une solution de collagène ou une solution de gélatine.

12. Procédé pour préparer un matériau à usage médical consistant :
(a) à stratifier avec un adhésif, deux feuilles de membrane de collagène ayant un matériau intermédiaire analogue à un treillis interposé entre elles, dans lequel la taille moyenne de pores du matériau intermédiaire analogue à un treillis est comprise entre 100 et 2 000 µm ;
(b) à maintenir le matériau stratifié résultant sous une pression réduite de sorte que les deux feuilles de membrane de collagène adhèrent l'une à l'autre à travers l'intermédiaire analogue à un treillis ; et
(c) à réticuler le matériau stratifié.

13. Procédé selon la revendication 12, dans lequel le matériau est soumis ensuite à un traitement par succinylation.

14. Procédé selon la revendication 12, dans lequel la membrane de collagène est un collagène solubilisé avec une base ou un collagène solubilisé avec une enzyme.

15. Procédé selon la revendication 12, dans lequel la membrane de collagène provient d'un amnios humain ou d'un chorion humain.

16. Procédé selon la revendication 15, dans lequel le matériau intermédiaire analogue à un treillis est interposé avec deux feuilles de la membrane de collagène d'un amnios humain de sorte que leurs surfaces sont dirigées vers un foetus se trouvent à l'extérieur.

17. Procédé selon la revendication 15, dans lequel le matériau intermédiaire analogue à un treillis est interposé avec deux feuilles de la membrane de collagène d'un amnios humain de sorte que leurs surfaces dirigées vers un chorion se trouvent à l'extérieur.

18. Procédé selon la revendication 12, dans lequel le diamètre moyen des pores du matériau intermédiaire analogue à un treillis est de 100 à 2 000 µm.

19. Procédé selon la revendication 12, dans lequel le matériau intermédiaire analogue à un treillis est dégradable et absorbable dans un corps vivant.

20. Procédé selon la revendication 12, dans lequel le matériau intermédiaire analogue à un treillis n'est pas dégradable et n'est pas absorbable dans un corps vivant.

21. Procédé selon la revendication 19, dans lequel le matériau intermédiaire est un poly(acide glycolique), un poly(acide lactique), un copolymère de l'acide glycolique et de l'acide lactique, une polydioxanone, un copolymère de l'acide glycolique et du carbonate de triméthylène ou un mélange d'un poly(acide glycolique) et d'un poly(acide lactique).

22. Procédé selon la revendication 20, dans lequel le matériau intermédiaire est un silicone, un polytétrafluoroéthylène, un polyéthylène, un polypropylène, un poly(téréphtalate d'éthylène), un polyuréthanne, un poly(alcool vinylique) ou un nylon.

23. Procédé selon la revendication 12, dans lequel l'adhésif est une solution de collagène ou une solution de gélatine.
